(19) Europäisches Patentamt
European Patent Office
Office européen des brevets

(11) Veröffentlichungsnummer: **0 004 582**
**B1**

(12) # EUROPÄISCHE PATENTSCHRIFT

(45) Veröffentlichungstag der Patentschrift: **01.07.81**

(51) Int. Cl.³: **C 07 D 263/44**

(21) Anmeldenummer: **79100782.6**

(22) Anmeldetag: **15.03.79**

(54) **Verfahren zur Herstellung von Oxazolidin-2,4-dionen.**

(30) Priorität: **31.03.78 DE 2813873**

(43) Veröffentlichungstag der Anmeldung: **17.10.79 Patentblatt 79/21**

(45) Bekanntmachung des Hinweises auf die Patenterteilung: **01.07.81 Patentblatt 81/26**

(84) Benannte Vertragsstaaten: **BE CH DE FR GB IT LU NL SE**

(56) Entgegenhaltungen: **DE-C-729 850**
**GB-A-982 940**

(73) Patentinhaber: **BASF Aktiengesellschaft, Carl-Bosch-Strasse 38, D-6700 Ludwigshafen (DE)**

(72) Erfinder: **Scholz, Herbert, Dr., Londoner Ring 7, D-6700 Ludwigshafen (DE)**

**EP 0 004 582 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Oxazolidin-2,4-dionen

Die Erfindung betrifft ein neues Verfahren zur Herstellung von Oxazolidin-2,4-dionen durch Umsetzung von Carbamaten mit 2-Hydroxycarbonsäureestern.

Es ist bekannt, Oxazolidin-2,4-dione durch Umsetzung von Isocyanaten mit 2-Hydroxycarbonsäureestern herzustellen (DE-AS 18 11 843, DE-OS 20 22 494, DE-OS 22 07 576).

Es wurde nun gefunden, daß man ein Oxazolidin-2,4-dion der Formel

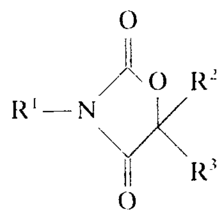

in der $R^1$

einen gegebenenfalls durch ein oder mehrere Halogenatome, Methyl oder Methoxy substituierter Arylrest mit 6 bis 12 Kohlenstoffatomen oder einen Alkylrest mit 1 bis 4 C-Atomen oder einen Allylrest,

$R^2$ Wasserstoff oder einen Alkylrest mit 1 bis 4 C-Atomen oder einen Methoxymethylrest oder einen Vinylrest bedeutet und

$R^3$ die gleichen Bedeutungen wie $R^2$ hat, wobei $R^2$ und $R^3$ im Einzelfall gleich oder verschieden sind, in guter Ausbeute und großer Reinheit erhält, wenn man

ein Carbamat der Formel

$$R^1—NH—COOR^4$$

in der

$R^1$ die obengenannte Bedeutung hat und

$R^4$ einen Alkylrest mit 1 bis 10 C-Atomen oder einen Cyclohexylrest oder einen Phenylrest bedeutet, bei einer Temperatur zwischen 80 und 250°C, gegebenenfalls in Gegenwart eines Katalysators mit einem 2-Hydroxycarbonsäureester der Formel

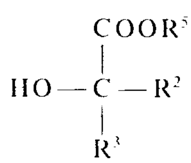

umsetzt, in der

$R^5$ die gleichen Bedeutungen wie $R^4$ hat und
$R^4$ und $R^5$ im Einzelfall gleich oder verschieden sind und
$R^2$ und $R^3$ die obengenannten Bedeutungen haben.

Die verschiedenen Substituenten haben beispielsweise die folgenden Bedeutungen:

$R^1$ Aryl, z. B. Phenyl, Naphthyl, 2-, 3- und 4-Fluorphenyl, 2-, 3- und 4-Chlorphenyl, 2-, 3- und 4-Bromphenyl, 2,3-Dichlorphenyl, 2,3,6-Trichlorphenyl, 2-, 3- und 4-Methoxyphenyl, 2-, 3- und 4-Methylphenyl, 3,4-Dichlorphenyl, 2,6-Dichlorphenyl, 2,3,6-Trichlorphenyl, 3,4,5-Trichlorphenyl, 2,4,6-Trichlorphenyl, 2,3,5-Trichlorphenyl und bevorzugt 3,5-Dichlorphenyl
oder Alkyl z. B. Methyl, Äthyl, Isopropyl
oder Allyl,

$R^4$ Alkyl z. B. Methyl, Isobutyl, Butyl, Propyl, Äthyl; Cyclohexyl oder Phenyl, $R^2$ Wasserstoff oder Vinyl oder Alkyl z. B. Methyl, Äthyl.

Bevorzugte Reste sind

| | |
|---|---|
| $R^1$ | 3,5-Dichlorphenyl, |
| $R^4$ und $R^5$ | Isobutyl, Methyl, Butyl, |
| $R^2$ | Methyl und |
| $R^3$ | Vinyl. |

Für die Durchführung der Umsetzung ist eine erhöhte Temperatur zwischen 80 und 250°C, vorzugsweise zwischen 120 und 225°C erforderlich. Weiterhin ist es von Vorteil in Gegenwart eines Katalysators zu arbeiten, weil dadurch die Temperatur der Umsetzung erniedrigt werden kann.

Als Katalysatoren werden z. B. Halogenide, Oxide oder Carboxylate mit 1 bis 12 C-Atomen von Metallen wie Zinn, Blei, Kobalt oder Kupfer verwendet. Beispielsweise können Bleiacetat, Kupfer-II-acetat, Kobaltchlorid, Dibutylzinndilaurat oder Dibutylzinnoxid als Katalysatoren eingesetzt werden.

Vorzugsweise werden jedoch tertiäre organische Amine wie Trimethylamin, Triäthylamin, Tripropylamin, Tributylamin, Tripentylamin, Trihexylamin, Triheptylamin, Trioctylamin, Trinonylamin, Tridecylamin, Dimethylcyclohexylamin, Dimethylanilin als Katalysatoren verwendet.

Die Umsetzung kann ohne Lösungsmittel durchgeführt werden; der Zusatz von Lösungsmitteln kann vorteilhaft sein, insbesondere wenn die Lösungsmittel mit den abzudestillierenden Alkoholen $R^4$ OH und $R^5$ OH Azeotrope bilden ohne mit dem Ausgangsmaterial 2-Hydroxycarbonsäureester ein Azeotrop zu bilden.

Lösungsmittel sind z. B. Xylol, Benzol, α-Pinen, Methylisobutylketon, Isobutylacetat, Cyclohexan oder vorzugsweise Toluol.

Für die Umsetzung werden vorteilhaft stöchiometrische Mengen der Ausgangsprodukte verwendet. Es ist jedoch auch möglich, einen Überschuß, beispielsweise bis zu 10%, an einem der Ausgangsprodukte, zweckmäßig an dem billigeren Ausgangsprodukt, zu verwenden.

Wichtig für eine gute Ausbeute bei der Umsetzung ist es, die entstehenden Alkohole $R^4$ OH und $R^5$ OH möglichst quantitativ abzutrennen, vorzugsweise abzudestillieren und dafür zu sorgen, daß der als Ausgangsprodukt verwendete 2-Hydroxycarbonsäureester nicht mitdestilliert. Zu diesem Zweck ist die Verwendung von Destillationskolonnen mit Füllkörpern besonders vorteilhaft.

Die Umsetzung kann bei Normaldruck durchgeführt werden; ein schwaches Vakuum von 65 bis 950 mbar kann von Vorteil sein, um die Destillationstemperatur zu erniedrigen.

Die als Ausgangsprodukte verwendeten Carbamate erhält man durch Umsetzung eines Isocyanats mit Alkohol oder eines Carbaminsäurechlorids mit Alkohol in Gegenwart von Alkali in üblicher Weise. So erhält man beispielsweise durch Umsetzung von 3,5-Dichlorphenylisocyanat mit Methylalkohol den N-(3,5-Dichlorphenyl)-carbaminsäuremethylester (Fp: 121°C).

Verwendet man anstelle von Methylalkohol den Isobutylalkohol, dann erhält man den N-(3,5-Dichlorphenyl)-carbaminsäureisobutylester (Fp: 75°C). Aus Methylisocyanat und Methylalkohol erhält man den N-Methylcarbaminsäuremethylester.

Die folgenden Beispiele erläutern die Durchführung des erfindungsgemäßen Verfahrens.

## Beispiel 1

132 Teile (Gewichtsteile) (0,6 mol) N-(3,5-Dichlorphenyl)-methylcarbamat (Fp: 121°C), 105,3 Teile (0,6 mol) 98%iger Vinylmilchsäureisobutylester und 5,6 Teile (0,03 mol) Tributylamin werden gemischt. Über eine 40 cm hohe Destillationskolonne (mit 2 cm Durchmesser gefüllt mit Maschendrahtringen mit 2400 Maschen/$cm^2$ aus nichtrostendem Stahl) werden im Laufe von 95 Minuten bei einem Druck von 200 mbar bei einer Temperatur der Mischung von 145 bis 171°C und einer Dampftemperatur von 43 bis 61,5°C 38,4 Teile Destillat (Methanol und Isobutanol) abdestilliert. Der Rückstand wird auf 90°C abgekühlt (er bleibt flüssig) und unter Rühren in 150 Teilen Methanol von 20°C getropft. Man rührt 2 Stunden bei 20°C nach, saugt das abgeschiedene Festprodukt ab und wäscht zweimal mit je 20 Teilen Methanol.

Ausbeute: 150 Teile 3-(3,5-Dichlorphenyl)-5-methyl-5-vinyl-1,3-oxazolidin-2,4-dion
(Vinclozolin) (=88% Ausbeute)
Fp: 106—110°C.

## Beispiel 2

159,2 Teile (0,6 mol) N-(3,5-Dichlorphenyl)-isobutylcarbamat (Fp: 75°C) und 105,3 Teile (0,6 mol) 98%iger Vinylmilchsäureisobutylester werden mit 5,6 Teilen (0,03 mol) Tributylamin 1 Stunde unter Rückfluß erhitzt. Über eine Destillationskolonne (Daten wie im Beispiel 1) werden im Laufe von 5 Stunden bei einer Temperatur der Mischung von 198 bis 220°C und einer Dampftemperatur von 102°C

bis 107° C 73 Volumenteile = 57,8 Gewichtsteile Destillat (Isobutanol) abdestilliert. Den Rückstand läßt man auf 80° C abkühlen (er bleibt flüssig) und tropft ihn in 150 Teilen Methanol bei 20° C. Es wird 2 Stunden bei 20° C nachgerührt. Das abgeschiedene Vinclozolin wird abgesaugt und getrocknet.

Ausbeute: 121 Teile Vinclozolin ≙ 70,5%
Fp.: 105° C.

Beispiel 3

67 g-Teile (0,76 mol) Methylcarbamidsäuremethylester, 114 Volumenteile Toluol, 100 Teile (0,76 mol) 2-Hydroxyisobuttersäureäthylester und 28 Teile (0,152 mol) Tributylamin werden 1 Stunde unter Rückfluß erhitzt. Über eine Destillationskolonne (Daten Beispiel 1) werden Toluol, Methanol und Äthanol abdestilliert (Temperatur des Gemisches 155 bis 190° C, Dampftemperatur 79 bis 90° C). Der Destillationsrückstand wird bei 10 mbar fraktioniert destilliert.

Bei 87,5° C geht 3-N-Methyl-5,5-dimethyl-1,3-oxazolidinon-2,4 über, das auskristallisiert. Fp: 44° C.

Die folgenden Beispiele 4 bis 10 wurden analog zu dem Beispiel 1 durchgeführt und verlaufen nach folgendem Reaktionsschema:

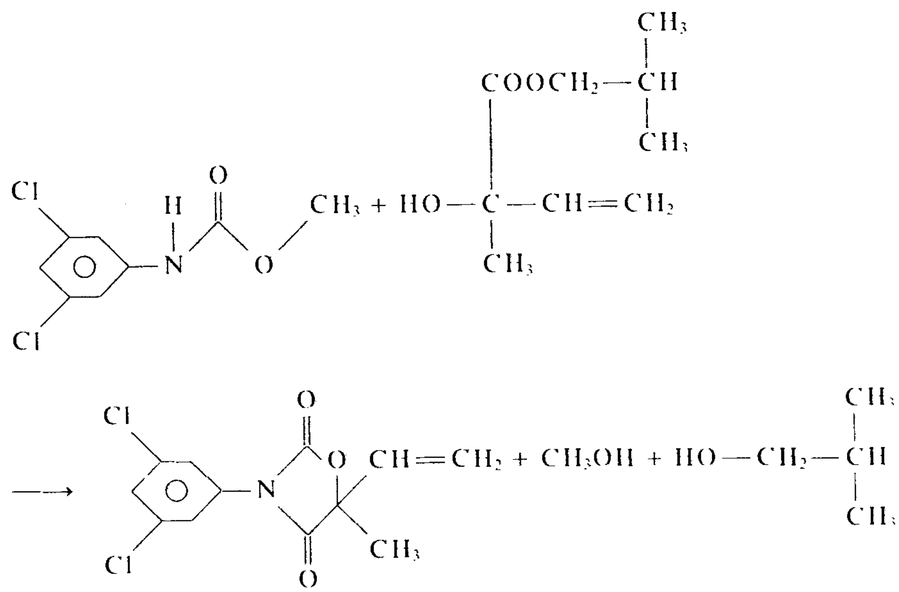

| Beispiel Nr. | Ansatz (mol) | Katalysator Art | Menge (mol) | Ausbeute Vinclozolin % | Temperatur des Gemisches °C | Reaktionszeit (Stunden) | Bemerkungen |
|---|---|---|---|---|---|---|---|
| 4 | 0,6 | Dibutylzinndilaurat | 0,06 | 93,5 | 160–190 | 2 | |
| 5 | 0,6 | ohne | – | 73 | 200–225 | 2 | |
| 6 | 0,6 | Kupfer-II-acetat | 0,06 | 76 | 160–190 | 2 | mit Wasser gewaschen |
| 7 | 0,6 | Trioctylamin | 0,06 | 93,5 | 150–200 | 1 | |
| 8 | 0,6 | Kobalt-II-chlorid | 0,06 | 82,5 | 165–195 | 2 | |
| 9 | 0,6 | Blei-II-acetat | 0,06 | 91 | 160–200 | 1 | mit Wasser gewaschen |
| 10 | 0,6 | Dibutylzinnoxid | 0,06 | 88 | 135–185 | 3 | |

**Patentansprüche**

1. Verfahren zur Herstellung von Oxazolidin-2,4-dionen der Formel

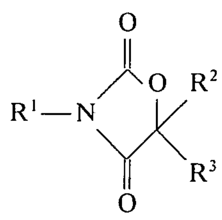

in der

$R^1$ einen gegebenenfalls durch ein oder mehrere Halogenatome, Methyl oder Methoxy substituierter Arylrest mit 6 bis 12 Kohlenstoffatomen oder einen Alkylrest mit 1 bis 4 C-Atomen oder einen Allylrest,
$R^2$ Wasserstoff oder einen Alkylrest mit 1 bis 4 C-Atomen oder einen Methoxymethylrest oder einen Vinylrest bedeutet und
$R^3$ die gleichen Bedeutungen wie $R^2$ hat, wobei $R^2$ und $R^3$ im Einzelfall gleich oder verschieden sind,

dadurch gekennzeichnet, daß man ein Carbamat der Formel

$$R^1—NH—COOR^4$$

in der

$R^1$ die obengenannten Bedeutungen hat und
$R^4$ einen Alkylrest mit 1 bis 10 C-Atomen oder einen Cyclohexylrest oder einen Phenylrest bedeutet, bei einer Temperatur zwischen 80 und 250°C, gegebenenfalls in Gegenwart eines Katalysators, mit einem 2-Hydroxycarbonsäureester der Formel

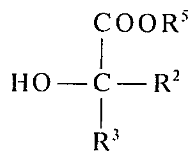

umsetzt, in der

| | |
|---|---|
| $R^5$ | die gleichen Bedeutungen wie $R^4$ hat und |
| $R^4$ und $R^5$ | im Einzelfall gleich oder verschieden sind und |
| $R^2$ und $R^3$ | die obengenannten Bedeutungen haben. |

2. Verfahren gemäß Anspruch 1, dadurch gekennzeichnet, daß man als Katalysator ein tertiäres Amin verwendet.
3. Verfahren gemäß Anspruch 1, dadurch gekennzeichnet, daß man die bei der Umsetzung entstehenden Alkohole $R^4$ OH und $R^5$ OH abtrennt, vorzugsweise abdestilliert.
4. Verfahren gemäß Anspruch 1, dadurch gekennzeichnet, daß man die Umsetzung mit Verbindungen durchführt, bei denen $R^1$ 3,5-Dichlorphenyl, $R^2$ Methyl und $R^3$ Vinyl bedeuten.

**Claims**

1. A process for the preparation of an oxazolidine-2,4-dione of the formula

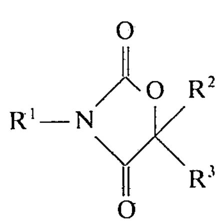

where

$R^1$ is aryl of 6 to 12 carbon atoms which is unsubstituted or substituted by one or more halogen atoms, methyl or methoxy or is alkyl of 1 to 4 carbon atoms or allyl,
$R^2$ is hydrogen or alkyl of 1 to 4 carbon atoms or vinyl, and
$R^3$ has the same meanings as $R^2$, and $R^2$ and $R^3$ may be identical or different,

characterized in that a carbamate of the formula

$$R^1—NH—COOR^4$$

where

$R^1$ has the above meanings and
$R^4$ is alkyl of 1 to 10 carbon atoms or cyclohexyl or phenyl, is reacted with a 2-hydroxycarboxylic acid ester of the formula

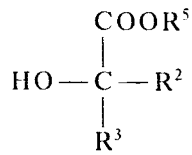

where

$R^5$ has the same meanings as $R^4$,
$R^4$ and $R^5$ may be identical or different and
$R^2$ and $R^3$ have the above meanings, at from 80 to 250° C, in the presence or absence of a catalyst.

2. A process as claimed in claim 1, characterized in that a tertiary amine is used as catalyst.
3. A process as claimed in claim 1, characterized in that the alcohols $R^4OH$ and $R^5OH$ formed in the reaction are separated off, preferably distilled off.
4. A process as claimed in claim 1, characterized in that the reaction is carried out with a compound where $R^1$ is 3,5-dichlorophenyl, $R^2$ is methyl and $R^3$ is vinyl.

**Revendications**

1. Procédé de préparation d'oxazolidin-2,4-diones de formule

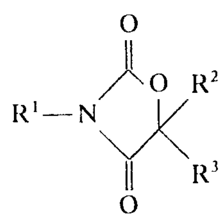

dans laquelle

$R^1$ est un reste aryle en $C_6$ à $C_{12}$ éventuellement substitué par un ou plusieurs atomes d'halogène, groupes méthyle ou méthoxy, ou un reste alkyle en $C_1$ à $C_4$ ou un reste allyle,
$R^2$ est l'hydrogène ou un reste alkyle en $C_1$ à $C_4$ ou un reste méthoxyméthyle ou un reste vinyle et
$R^3$ a la même signification que $R^2$, $R^2$ et $R^3$ étant identiques ou différents,

caractérisé par le fait que l'on fait réagier, à une température comprise entre 80 et 250°C, éventuellement en présence d'un catalyseur, un carbamate de formule

$$R^1—NH—COOR^4$$

dans laquelle

$R^1$ a la signification indiquée plus haut et

$R^4$ est un reste alkyle en $C_1$ à $C_{10}$ ou un reste cyclohexyle ou un reste phényle, avec un ester d'acide 2-hydroxycarboxylique de formule

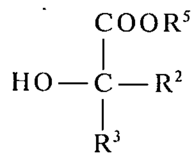

dans laquelle

$R^5$ a la même signification que $R^4$ et
$R^4$ et $R^5$ sont identiques ou différents et
$R^2$ et $R^3$ ont les significations données plus haut.

2. Procédé selon la revendication 1, caractérisé par le fait que l'on utilise comme catalyseur une amine tertiaire.

3. Procédé selon la revendication 1, caractérisé par le fait que l'on sépare, de préférence par distillation, les alcools $R^4$ OH et $R^5$ OH produits lors de la réaction.

4. Procédé selon la revendication 1, caractérisé par le fait que l'on effectue la réaction avec des composés dans lesquels $R^1$ représente 3,5-dichlorophényle, $R^2$ méthyle et $R^3$ vinyle.